# EUROPEAN PATENT SPECIFICATION

(11) **EP 3 867 366 B1**
(45) Date of publication and mention of the grant of the patent: **30.04.2025**
(21) Application number: 19805382.9
(22) Date of filing: 15.10.2019
(51) Int. Cl.: C12N 9/22, C12N 15/10, C12N 15/63

(54) **CRISPR SYSTEM FOR GENOME EDITING**
KRISPR-SYSTEM ZUR GENOMEDITIERUNG
SYSTÈME CRISPR POUR ÉDITION GÉNOMIQUE

(30) Priority: 15.10.2018 IT 201800009431
(43) Date of publication of application: 25.08.2021
(73) Proprietor: Universita' Degli Studi di Siena, 53100 Siena (IT); ISTITUTO PER LO STUDIO, LA PREVENZIONE E LA RETE ONCOLOGICA (ISPRO), 50139 Firenze (IT); Universita' Degli Studi di Firenze, 50121 Firenze (IT)
(72) Inventor: RENIERI, Alessandra, 53100 Siena (IT); CONTICELLO, Silvestro, 50134 Firenze (IT); DONATI, Francesco, 52100 Arezzo (IT); NICCHERI, Francesca, 50022 Greve In Chianti ( Firenze) (IT); MARI, Francesca, 53100 Siena (IT); PAPA, Filomena Tiziana, 53100 Siena (IT); LORENZETTI, Flaminia Clelia, 3000 Leuven (BE)
(74) Representative: Rimini, Rebecca
(86) International application number: PCT/IB2019/058760
(87) International publication number: WO 2020/079574

(56) References cited:
- WO-A1-2016/094888
- WO-A1-2018/009525
- ZHANG-HUI CHEN ET AL: "Targeting genomic rearrangements in tumor cells through Cas9-mediated insertion of a suicide gene", NATURE BIOTECHNOLOGY, vol. 35, no. 6, 1 May 2017 (2017-05-01), New York, pages 543 - 550, XP055573309, ISSN: 1087-0156, DOI: 10.1038/nbt.3843
- CHRISTOPHER A. LINO ET AL: "Delivering CRISPR: a review of the challenges and approaches", DRUG DELIVERY., vol. 25, no. 1, 1 January 2018 (2018-01-01), US, pages 1234 - 1257, XP055573342, ISSN: 1071-7544, DOI: 10.1080/10717544.2018.1474964
- CIA-HIN LAU ET AL: "In vivo genome editing in animals using AAV-CRISPR system: applications to translational research of human disease", F1000RESEARCH, vol. 6, 1 January 2017 (2017-01-01), pages 2153, XP055573337, DOI: 10.12688/f1000research.11243.1
- MARTA MARTINEZ-LAGE ET AL: "CRISPR/Cas9 for Cancer Therapy: Hopes and Challenges", BIOMEDICINES, vol. 6, no. 4, 12 November 2018 (2018-11-12), pages 105, XP055573345, DOI: 10.3390/biomedicines6040105

## Description

The present invention relates to a system which can be used to make changes within a cell genome. In particular, the invention relates to a "Clustered Regularly Interspersed Short Palindromic Repeat (CRISPR)-CRISPR associated (Cas) (CRISPR-Cas)" system targeting a genomic target sequence in a eukaryotic cell, as well as a viral particle comprising said system. The invention further relates to a method employing the aforementioned CRISPR-Cas system or the aforementioned viral particle.

In recent decades, thanks to the remarkable progress in molecular biology and the sequencing of numerous genomes, important goals have been achieved in the field of genome editing technologies, that is, the combination of methods that allow for changes in the genome.

Currently, the Cluster Regularly Interspaced Short Palindromic Repeats (CRISPR) system is one of the most advanced methods for making targeted changes in the genome of target cells. CRISPR technology is capable of cutting double-stranded DNA by using an RNA molecule, called guide RNA (gRNA), which guides an endonuclease enzyme to a specific target site on the genome. Among the most commonly used endonuclease enzymes are Cas9 and Cas12a (formerly referred to as Cpf1) which differ essentially in the type of double-strand breakage produced at the target sites. Cas9 enzyme makes double-stranded cuts leaving blunt ends, which are usually repaired in the cell by a non-homologous binding mechanism, while Cas12a enzyme produces cohesive ends that facilitate homology directed repair in the cell (Zetsche B. et al, "Cpf1 is a single RNA-guided endonuclease of a class 2 CRISPR-Cas system"; (2015) Cell, 163(3):759-71). More precisely, homologous repair is a DNA repair mechanism based on the possibility of exchanging genetic material between DNA fragments that have regions with identical or very similar nucleotide sequences. Generally, non-homologous repair is chosen to obtain gene knock-out (KO), i.e. suppression of the expression of a given gene, since this repair mode generates insertions and deletions (indels) at the target site, while homology directed repair is preferred for knock-in (KI) experiments in order to integrate the desired nucleotide sequence into the specific genomic locus.

Recently, the CRISPR system entered the field of therapy, with the first CRISPR trial in humans (NCT02793856) beginning in 2016 at the Sichuan University's West China Hospital in Chengdu (China) for the treatment of metastatic non-small cell lung carcinoma, a disease in which treatments such as chemotherapy and radiotherapy did not produce significant effects. In the treatment of recurrent and refractory acute lymphocytic leukemia, the CRISPR system has also been used in *ex vivo* T cell genetic engineering in order to produce chimeric antigen receptors (CARs) targeting specific surface molecules on tumour cells. Two further clinical trials related to the CRISPR system are currently under way, both started in June 2017. One of the aforementioned trials (NCT03166878) is in phase II and is based on the use of CAR-T cells against CD19 antigen in patients with recurrent or refractory B-cell leukemia or lymphoma. The second trial (NCT02706392), still in phase I, is using CAR-T cells directed against ROR1 (Receptor tyrosine kinase-like orphan receptor) in patients with ROR1+ chronic lymphocytic leukemia, mantle cell lymphoma (MCL), acute lymphocytic leukemia, Phase IV non-small cell lung cancer (NSCLC) or metastatic triple-negative breast cancer (TNBC).

Therefore, the field of neoplastic diseases plays a primary role among therapeutic applications of CRISPR technology.

Typically, during its evolution, the tumour acquires new and particular genetic mutations that become specific markers of the disease, thereby allowing a particular type of tumour and its molecular progress to be identified. Thus, a certain gene mutation specifically associated with a particular tumour can be targeted through a personalized therapy. Among neoplastic transformation target genes, one of the most affected is the *TP53* gene which encodes a protein, called p53, which plays different roles in the human cell. The canonical function of *TP53* as a tumour suppressor gene has been known for many years, however over-expression of the mutant *TP53* gene, through the gain-of-function mechanism, results in the reversal of its role into an oncogene (non-canonical function).

*TP53* mutations in haematological and solid tumours have been identified at different levels depending on the types and subtypes of cancer. Generally, *TP53* mutations are detected in case of relapse, which leads to an aggressive and often refractory disease course, with poor therapeutic response of the patient. From a therapeutic point of view, *TP53* gene is therefore considered as an important target for treatment, especially when other types of pharmacological treatments are not effective.

B-cell chronic lymphocytic leukemia (B-CLL) is the most common model of leukemia in adults. Modifications in the p53 protein pathway are frequently described in B-CLL and are mainly associated with chromosome 17p13 deletion or *TP53* gene mutations. *TP53* gene mutations are found in approximately 10% of patients with B-CLL and other haematologic and non-haematologic malignancies, and the average 5-year survival rate in these patients is estimated at around 20% compared to a 70% survival rate reported in the other patients with B-CLL.

In recent years, genome editing studies have focused in particular on mutagenesis experiments designed to achieve the replacement of mutated gene sequences with the respective correct DNA sequences. Cells repair DNA double-strand damage more easily by using non-homologous recombination than homologous repair since the latter mechanism is mainly active during the S and G2 phases of the cell cycle, whereas non-homologous repair can occur in any phase of the cell cycle. The prevalence in cells of non-homologous DNA repair mechanisms therefore leads to great difficulties in mutagenesis applications. A different strategy was followed by directing the genome editing methodology towards integrating a specific foreign nucleotide sequence into the target genomic site, and not towards correcting a DNA sequence mutation. Such an approach is illustrated in the article by Chen ZH and colleagues, which describes the use of a CRISPR system to integrate a sequence encoding a viral enzyme into the genome of a tumour cell (Chen ZH, et al, "Targeting genomic rearrangements in tumor cells through Cas9-mediated insertion of a suicide gene", (2017) Nat Biotechnol. 35:543-550). In particular, Chen ZH et al describe a CRISPR-Cas-mediated insertion of Herpes Simplex Virus thymidine kinase into the chromosomal breakpoints in human tumour xenografts in mice. Treatment of these tumours with ganciclovir after introduction of thymidine kinase led to cell death and to a reduction of tumour size and mortality.

WO2016094888A1 discloses CRISPR-Cas gene editing for introducing a suicidal gene in a cancer-specific fusion gene, thereby leading to the selective elimination of cancer cells without affecting normal cells.

WO2018009525 discloses a cancer treatment that involves the use of a CRISPR-Cas system comprising a bidirectional promoter, a genome targeted nuclease (Cas9 protein) and guide RNAs directed towards oncogenes or tumour suppressor genes, which is packaged in a compact adeno-associated virus (AAV) particle. However, WO2018009525 is silent as to the use of a nucleotide sequence encoding a protein capable of promoting cell death.

Despite the remarkable progress achieved by genome modification methods, and in particular by CRISPR technology, their application suffers from major limitations mainly attributable to the failure to achieve adequate accuracy of their mechanisms of action. More particularly, one of the major problems associated with the CRISPR system is the possibility that the guide RNA molecule used in this method might recognize on the genome not only the sequence to be modified, but also sequences very similar thereto, resulting in modification of a genomic locus different from the target. This occurrence, referred to as off-target, could lead, for example, to undesired modifications in a tumour suppressor gene, thus triggering uncontrolled reproduction of the target cells and their possible neoplastic transformation.

In addition to the inconvenience of the possible off-target activity, the CRISPR technique also poses the problem of directing the transfer of its components exclusively within the cell population in which the genetic modification is to be performed, for example a particular population of diseased cells, so as to avoid affecting healthy cells that do not require genetic manipulation. To this end, different methods have been evaluated to allow the correct release of the CRISPR system in target cells, including chemical systems (lipofectamine, polyethyleneimine) or electroporation. However, these methods work efficiently mainly in *in vitro* or *ex vivo* studies, whereas the only currently available approaches for CRISPR release in *in vivo* experiments are viral systems which, in the formats in which they are currently used, are still associated with low safety.

The use of the CRISPR technology to integrate a foreign coding nucleotide sequence into the cellular genome also raises the problem of preserving the expression of said sequence when the target genomic site carries a nonsense mutation which, by leading to a stop codon, could cause the translation of the protein encoded by the foreign sequence to stop.

Therefore, there is a need to provide genome editing systems, in particular CRISPR systems, which are provided with a high degree of accuracy and precision, thereby making these systems suitable for use in clinical and therapeutic settings. More particularly, there is a need to provide CRISPR systems which are able to selectively operate on target cells, without affecting normal cells in any way, and at the same time capable of reducing exposure of cells to endonuclease activity, thereby abolishing or significantly reducing the occurrence of dangerous off-target events.

Furthermore, there is a need to provide CRISPR systems which allow genome editing operations such as to achieve insertion and correct expression of foreign nucleotide sequences even at mutated target genome sequences.

This and other needs are met by the CRISPR system of the invention as defined in the appended claim 1.

The invention also relates to a viral particle and an isolated eukaryotic cell comprising the CRISPR system of the invention, a method that uses said system, and the therapeutic use thereof.

Further features and advantages of the invention are identified in the appended claims and illustrated in detail in the following description.

The appended independent and dependent claims form an integral part of the present description.

As will be apparent from the following detailed description, the present invention provides a non-naturally occurring or engineered CRISPR system targeting at least one genomic target sequence in a target cell, which is defined by a combination of features suitable to provide said system with high precision and accuracy. The CRISPR system according to the invention is based on the use of a viral vector which allows intracellular expression of the various components of the aforementioned system. In general, a viral vector includes DNA or RNA sequences derived from a virus, which are designed to allow the virus to be packaged into a viral particle as well as to be transduced into a host cell. Preferably, the expression vector according to the present invention is a lentiviral, retroviral, adenoviral or adeno-associated viral vector, more preferably a lentiviral vector.

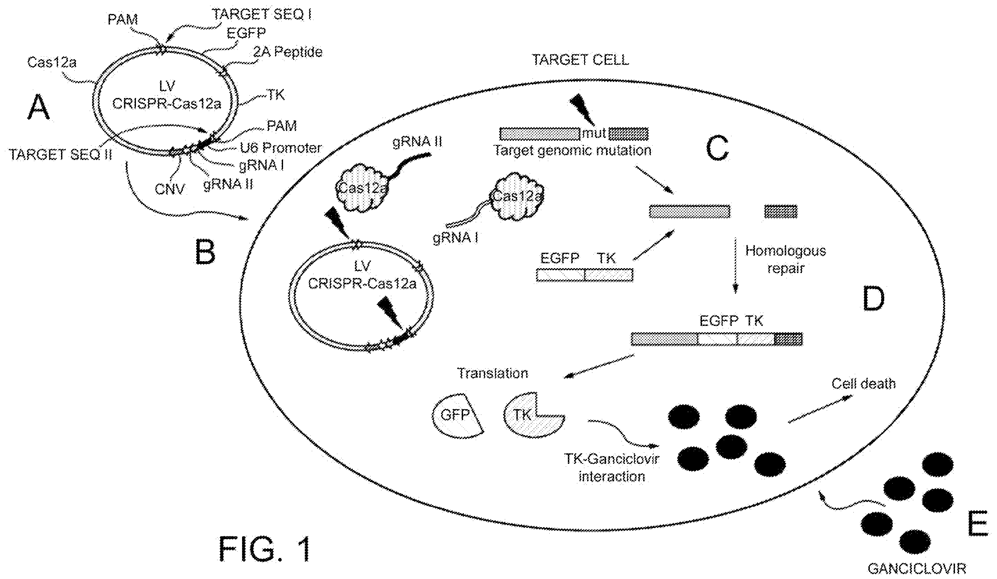

As previously mentioned and illustrated in panel A of Figure 1, the viral expression vector according to the present invention comprises nucleotide sequences encoding, respectively, the various components typically required for the CRISPR system to work, more specifically an endonuclease enzyme and one or more guide RNA molecules capable of binding said enzyme and directing it towards the target nucleotide sequences thanks to its ability to hybridize to the aforesaid sequences. Consequently, the endonuclease enzyme carried by the guide RNAs makes a double-stranded cut at the target sequences.

According to the present invention, the endonuclease enzyme is characterized by the ability to generate cohesive ends after cutting the double-stranded DNA. The presence of cohesive ends at a cleavage site advantageously allows DNA fragment insertion to be oriented, thereby maintaining the correct reading frame of the coding sequences.

Endonuclease enzymes suitable for use in the CRISPR system according to the invention include, for example, but are not limited to, Cas12a enzyme and mutants thereof capable of recognizing different "Protospacer Adjacent Motif" (PAM) nucleotide sequences. It is well known that PAM sequences are short sequences comprising from two to six base pairs required for the CRISPR systems to recognize the target sequence (Shah S. et al, "Protospacer recognition motifs Mixed identities and functional diversity (2013) RNA Biology 10(5): 891-899).

In a preferred embodiment, the endonuclease of the CRISPR system of the invention is Cas12a enzyme. Preferably, the endonuclease enzyme comprises one or more nuclear localization signals suitable for facilitating the transfer of the CRISP-Cas complex into the target cell nucleus.

The viral expression vector according to the invention further comprises two nucleotide sequences encoding a first and a second guide RNA (gRNA), respectively. The first gRNA comprises a first scaffold nucleotide sequence capable of binding the nuclease enzyme and a first guide nucleotide sequence capable of hybridizing to a first target nucleotide sequence. The second gRNA comprises a second scaffold nucleotide sequence capable of binding the endonuclease enzyme and a second guide nucleotide sequence capable of hybridizing to a second target nucleotide sequence.

Consequently, when expressed, the first and second gRNAs complex with the endonuclease enzyme, thus forming a first and a second CRISPR complex. The first and second guide sequences direct the sequence-specific bond of the first and second CRISPR complexes towards the respective first and second target sequences. As illustrated in greater detail in the following part, according to the present invention, a first and a second target sequence are located on the viral expression vector.

According to one embodiment, the scaffold sequences of the first and second gRNAs, respectively, may correspond to each other.

Within the scope of the present invention, the nucleotide sequences of the viral vector encoding the endonuclease enzyme and the first and second gRNAs are suitable for expression in a host cell since they are operably linked to one or more regulatory sequences. Regulatory sequences suitable for use in gene expression are known and described in the state of the art, therefore the selection and use thereof are well within the skills of those of ordinary skill in the art. Promoters, for example inducible promoters, as well as enhancers, internal ribosome entry sites and/or transcription termination signals are mentioned by way of non-limiting example. For example, the nucleotide sequences encoding the first and second gRNAs can be operably linked to a promoter that is recognized by eukaryotic RNA polymerase III, preferably a U6 promoter.

According to the present invention, the viral expression vector further comprises a nucleotide sequence encoding a protein capable of promoting the cell death process. Advantageously, said nucleotide sequence is not operably linked to any regulatory sequence located on the vector so that its expression is stably inhibited and only allowed when the nucleotide sequence is correctly integrated in a target genomic locus and equipped with the appropriate regulatory elements.

Exemplary proteins capable of promoting cell death include, but are not limited to, thymidine kinase, telomerases, cytosine deaminases, caspases, endonucleases, and specific cell death-inducing intracellular antibodies.

According to a more preferred embodiment, the protein capable of promoting cell death is a thymidine kinase (TK).

In the viral expression vector according to the invention, the target nucleotide sequences of the first and second gRNAs are also present, located downstream of the 3' end (first target sequence) and upstream of the 5' end (second target sequence), respectively, of the nucleotide sequence encoding the protein capable of promoting cell death. In order to allow the CRISPR system to recognise them with consequent endonuclease enzyme cleavage, both target nucleotide sequences are located downstream of the 3' end of a PAM sequence.

Therefore, the configuration of the elements of the viral expression vector, as previously illustrated, allows the excision of the sequence encoding the cell death-promoting protein from the vector by means of a sequence-specific enzymatic cleavage upstream and downstream of said sequence, thereby making the sequence available for insertion into a target genomic locus in a target cell. It should be noted that this feature introduces a further control element into the CRISPR system of the invention when transduced inside a target cell since the open conformation of the viral vector, as a result of endonuclease cleavage, facilitates its degradation and removal from the host cell, in particular from the cell nucleus. In this way, the possible permanence of the vector in the cell is prevented from leading to detrimental overexpression of the endonuclease enzyme encoded by the same, with consequent off-target cleavage of the DNA.

The CRISPR system according to the invention is also characterized in that the first target nucleotide sequence located on the viral expression vector corresponds to the at least one genomic target sequence of the eukaryotic cell targeted by the CRISPR system. Accordingly, the same gRNA, more specifically the first gRNA, is capable of directing endonuclease enzyme cleavage towards at least two distinct target sites - yet containing the same target sequence - one located on the viral expression vector and the other on the cell genome. Therefore, the DNA fragment containing the sequence encoding the cell death-promoting protein, when excised from the vector according to the method described above, can be inserted whilst retaining the reading frame at the cleavage site produced by the endonuclease enzyme at the at least one genomic target sequence targeted by the CRISPR system.

According to one embodiment, the first target nucleotide sequence and the second target nucleotide sequence located on the viral expression vector downstream of the 3' end and upstream of the 5' end, respectively, of the nucleotide sequence encoding the cell death-promoting protein are selected so as to be the same and have complementary single-stranded overhanging portions of the respective cohesive ends generated upon endonuclease cleavage.

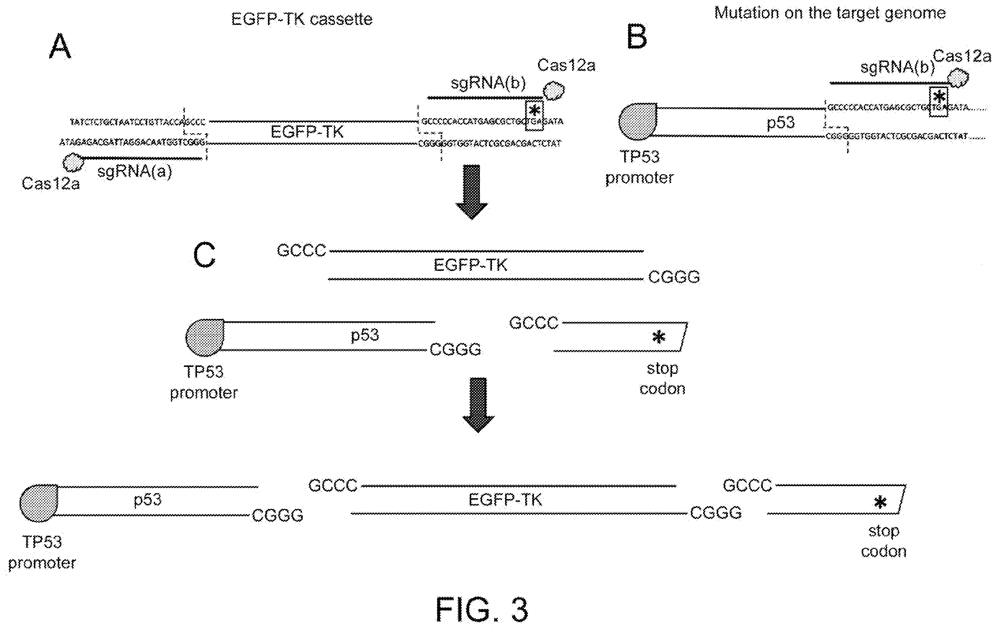

According to another embodiment, the first target nucleotide sequence and the second target nucleotide sequence located on the viral expression vector downstream of the 3' end and upstream of the 5' end, respectively, of the nucleotide sequence encoding the cell death-promoting protein are selected so as to be different and have complementary single-stranded overhanging portions of the respective cohesive ends generated upon endonuclease cleavage. This embodiment allows the use of a coding nucleotide sequence whose ends are different and is particularly suitable for applications in which the at least one genomic target sequence targeted by the CRISPR system of the invention contains a point mutation, for example c.548C>G (p.Ser183*), which generates a stop codon. In fact, in this circumstance, should a first and a second target nucleotide sequence, which are the same and correspond to the mutated genomic target sequence, be used in the CRISPR system of the invention, both the first and second target nucleotide sequences would consequently contain the nonsense mutation targeted by the CRISPR system. Therefore, the cleavage caused by the endonuclease, for example by Cas12a enzyme, on the genomic target sequence and the subsequent repair by insertion of the sequence encoding the cell death-promoting protein, whose excision from the vector was performed by using a first and a second target nucleotide sequence which were the same and both contained the nonsense mutation, would lead to the insertion of this mutation composition 5' of the coding sequence and would thus prevent the expression of the cell death-promoting protein. As illustrated by the diagram in Figure 3, the use of a first and a second target nucleotide sequence, which are different but have complementary single-stranded overhanging portions, allows the target nucleotide sequence to be modified 5' of the coding sequence so that it does not contain the stop codon. This ensures that, once the nucleotide sequence encoding the cell death-promoting protein has been inserted in the genomic target sequence, the only stop codon is present downstream of the coding sequence, thereby guaranteeing the correct expression of the cell death-promoting protein.

According to another embodiment, the CRISPR system of the invention targets a first and a second genomic target sequence. In this embodiment, the first genomic target sequence corresponds to the first target nucleotide sequence and the second genomic target sequence corresponds to the second target nucleotide sequence. This further facilitates the correct insertion of the DNA fragment containing the sequence encoding the cell death-promoting protein in the target genome since the cleavage mediated by each of the two gRNAs occurs once on the vector and once on the genome.

In one embodiment, the viral expression vector consists of nucleotide sequence SEQ ID NO.1.

According to a preferred embodiment, the viral expression vector of the system of the invention comprises a nucleotide sequence encoding a mutated integrase enzyme, in which the mutation causes inhibition of the enzymatic activity. The introduction of this mutation aims to increase the safety features of the CRISPR system of the invention as it prevents the integration of the viral vector into the genome of the host cell as well as the stable intracellular expression of the system components. This is particularly important for safeguarding normal, non-target cells if mistakenly transduced.

In a more preferred embodiment, the integrase enzyme encoded by the expression vector of the invention carries the point mutation p.Asp64Val.

The at least one genomic target sequence of the target cell targeted by the system may include one or more gene mutations. Gene mutations that may be present in the at least one genomic target sequence include, by way of non-limiting example, *TP53* gene mutations, *KRAS*/*BRAF* gene mutations, *NRAS* gene mutations, *EGFR* gene mutations in the region encoding the extracellular domain of the receptor, *HER2* gene amplification, *EML4*/*ALK* translocation, and *MET, PK3CA* and *ERB2* gene mutations, and any combination thereof.

Preferably, the mutation is a *TP53* gene mutation, more preferably a nonsense c.548C>G or p.Ser183* mutation, which causes an early stop of the translation, thus generating a truncated protein, or a nonsense c.818G>A or p.Arg273His mutation.

The target cell targeted by the CRISPR system according to the invention may be a eukaryotic cell, preferably a mammalian cell, more preferably a human cell, and even more preferably a human tumour cell.

Appropriate viral systems can be used, for example, to transfer the CRISPR system according to the invention into a target cell.

Therefore, a viral particle comprising a CRISPR system as defined above is also within the scope of the present invention. This viral particle can be, for example, a lentivirus, a retrovirus, an adenovirus, or an adeno-associated virus. Preferably, the viral particle is a lentivirus.

According to a preferred embodiment, the viral particle of the invention comprises a chimeric capsid protein comprising a domain capable of binding to at least one marker expressed on the surface of the target cell of the CRISPR system. The interaction between the chimeric protein domain as defined above and the cell surface marker ensures that the viral particle selectively targets the target cell, thereby avoiding the possibility of affecting and damaging healthy, non-target cells.

Preferably, the domain of the chimeric capsid protein is an antibody, a growth factor, or a ligand.

Proteins CD5, CD19, CD20, CD23, CD46 and BCR, and any combination thereof, are mentioned, for example, although not exclusively, among the cell surface markers.

An *in vitro* method for promoting cell death in a target cell, as defined in the appended claim 15, is also an object of the present invention. The method of the invention comprises transducing the target cell with a CRISPR system and/or at least one viral particle, as defined above, and culturing the transduced cell under suitable conditions for inducing the expression of the endonuclease enzyme and the first and second gRNAs. Typically, suitable culture conditions and times depend on the target cell and may be related, for example, to the composition of the culture medium, the pH, the relative humidity, the gaseous component of O₂ and CO₂, as well as the temperature. The selection of the most suitable culture conditions and times to be used in the method of the invention is well within the skills of those of ordinary skill in the art.

A first macromolecular complex including the endonuclease enzyme associated with the first gRNA and a second macromolecular complex including the endonuclease enzyme associated with the second gRNA form in the target cell as a result of the expression of the CRISPR system. As previously described and illustrated in Figure 1, these macromolecular complexes act by integrating the DNA insert encoding the cell death-promoting protein into the at least one genomic target sequence by means of sequence-specific cleavage on the viral vector and the cell genome at the first and second target sequences. More particularly, the cleavage at the first target sequence is operated by the first macromolecular complex by hybridization of the first gRNA to said first sequence, whereas the cleavage at the second target sequence is operated by the second macromolecular complex by hybridization of the second gRNA to said second sequence.

According to the method of the invention, the sequence encoding the cell death-promoting protein is expressed under conditions such that it is correctly inserted in the genomic target locus while maintaining the correct reading frame and under the control of suitable regulatory elements. This ensures that the succession of processes culminating in cell death is only triggered in the target cell.

Preferably, the integration of the DNA insert into the genome sequence is mediated by cellular DNA repair mechanisms, for example by ligation of DNA strand ends using microhomologous regions.

According to the embodiment shown in Figure 1, the protein capable of promoting cell death is the thymidine kinase (TK) enzyme. In this embodiment, the method according to the invention preferably further comprises the step of contacting the target cell with a substrate of the thymidine kinase, more preferably the ganciclovir substrate. The phosphorylation of this compound by thymidine kinase and the subsequent incorporation into the DNA molecule result in inhibition of the double helix synthesis, leading to cell death.

According to a more preferred embodiment, the target cell of the method of the invention is a diseased human cell, preferably a human tumour cell.

An isolated target cell comprising a CRISPR system or at least one viral particle as defined above also falls within the scope of the present invention.

Preferably, the isolated target cell is a eukaryotic cell, preferably a mammalian cell, more preferably a human cell, and even more preferably a human tumour cell.

As described above, the ability to operate in a targeted and selective manner on the genome of a target cell, triggering its cell death process, makes the CRISPR system of the invention particularly suitable for use in clinical-therapeutic applications, in particular in applications aiming at selectively removing the diseased cells.

Therefore, a further object of the present invention is the CRISPR system of the invention or the viral particle of the invention for use as a medicament. Preferably, but not exclusively, the therapeutic use is aimed at the treatment of a tumour disease, more preferably a disease selected from B-cell Chronic Lymphocytic Leukemia, non-Hodgkin lymphoma, myelodysplastic syndromes, myeloproliferative neoplasms, multiple myeloma, acute myeloid leukemia, acute lymphoblastic leukemia, lung squamous cell carcinoma, lung adenocarcinoma, ovarian cancer, colorectal cancer, esophageal cancer, head and neck cancer, laryngeal cancer, skin cancer, pancreatic cancer, stomach cancer, prostate cancer, liver cancer, brain tumour, bladder cancer, breast cancer, uterine cancer, soft tissue sarcoma, bone cancer, endocrine tumours and cervical cancer.

The experimental section that follows is provided for illustration purposes only and does not limit the scope of the invention as defined in the appended claims. In the experimental section, reference is made to the accompanying drawings, wherein:
Figure 1 is a schematic representation of the CRISPR system of the invention. (A) Representation of the structure of the viral expression vector according to the invention. (BE) Mechanism of action of the CRISPR system of the invention: (B) transduction of the viral expression vector into the target cell carrying the target genomic mutation; (C) (i) expression of Cas12a and of the two gRNAs (gRNA I and gRNA II), and binding of the latter to the endonuclease to form a first and a second macromolecular complex; (ii) transfer of the first and second macromolecular complexes towards the target sequences (TARGET SEQ I and TARGET SEQ II) present on the vector and the cell genome, and DNA cleavage at these sequences with excision of the sequence encoding the TK enzyme, optionally linked to the enhanced green fluorescent protein (EGFP); (D) insertion of the EGFP-TK-encoding sequence into the double-stranded cut on the genomic target sequence by homologous repair; (E) administration to the modified target cell of ganciclovir, which is converted by the TK enzyme into a toxic compound that causes the death of the cell.

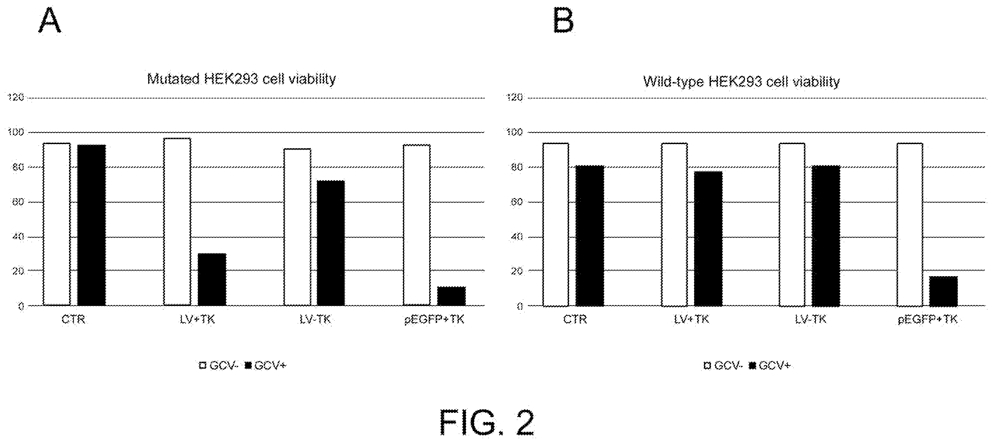

Figure 2 illustrates charts showing cell death figures, as determined by FACS analysis, in the presence (+) and the absence (-) of ganciclovir (GCV) in HEK293 cell cultures engineered with the target mutation (A) and in wild-type HEK293 cell cultures (B), transfected with the lentiviral vector (LV+TK), untransfected (CTR), transfected with the lentiviral vector devoid of the EGFP-TK cassette (LV-TK), and transfected with the lentiviral vector constitutively encoding the EGFP-TK sequence (pEGFP+TK). After 3 days in the presence or the absence of Ganciclovir treatment (GCV, 0.1 mg/ml) a 70% reduction in the number of cells in the culture was detected in the mutated HEK293 cell sample transfected with the CRISPR system of the invention, and an 80% reduction was detected in the mutated and wild type HEK293 cell samples transfected with the lentiviral vector constitutively encoding the EGFP-TK sequence.
Figure 3 is a schematic illustration of the cutting process performed by the enzyme Cas12a on the lentiviral vector and the target genome, showing that the mutation that introduces a stop codon remains after the correct integration of the EGFP-TK sequence (coding cassette) into the target genome 3' (downstream) of said coding sequence so as to allow the translation of the suicidal gene. Two different gRNAs are used to guide Cas12a enzyme to the target sequences. gRNA(a): arbitrary sequence specific for the 5' portion of the coding cassette; gRNA(b): mutated sequence specific for the mutated gene and the 3' region of the coding cassette. (A) detail of the EGFP-TK (coding cassette) sequence present in the lentiviral vector. The Cas12a enzyme cleavage site that produces cohesive ends is indicated by a dashed line; (B) detail of the TP53 genomic sequence containing the target mutation. The Cas12a enzyme cleavage site that produces cohesive ends is indicated by a dashed line. The TGA stop codon is boxed and indicated with an asterisk. (C) scheme of insertion of the coding cassette excised from the lentiviral vector by the Cas12a enzyme within the TP53 genomic sequence containing the target mutation.

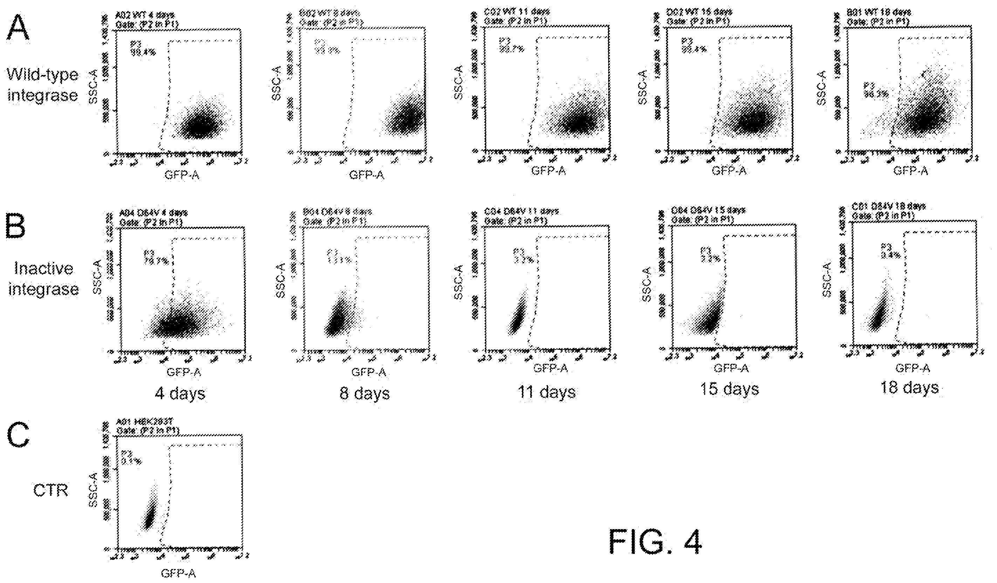

Figure 4 shows the results of HEK293T cell infection by wild-type integrases compared to transfection with inactive integrase. (A) HEK293T cells infected with the lentivirus characterized by wild-type integrases show unchanged expression of the GFP reporter protein 4, 8, 11, 15 and 18 days after infection. (B) On the other hand, HEK293T cells infected with the lentivirus characterized by inactive integrase show decreased expression of the GFP reporter protein already 8 days after infection, and its total clearance 11 days after infection, as in the untreated control (C).

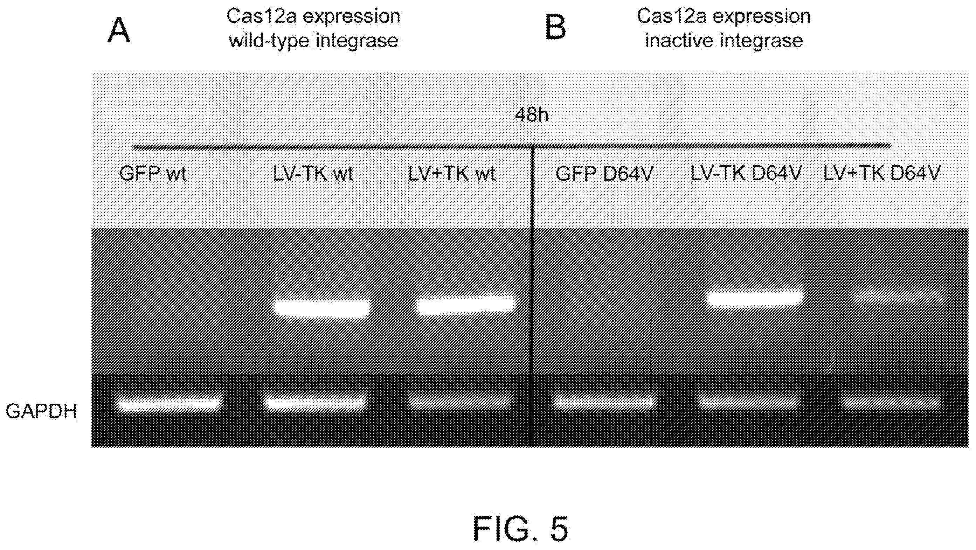

Figure 5 shows the results of the qPCR analysis carried out to test the expression of the Cas12a enzyme 48 hours after infection (A) of HEK293 cells with the virus carrying wild-type integrases compared to (B) the virus with the inactive integrase. LV-TK: lentiviral vector without the EGFP-TK cassette, where the target sequences leading to cassette
excision are absent. LV+TK: lentiviral vector with the EGFP-TK cassette carrying the target sequences leading to cassette excision. (A) No difference is detected in the expression of the Cas12a enzyme after infection with the wild-type integrase virus carrying LV-TK or LV+TK. (B) A significant difference in expression of the Cas12a enzyme is detected after infection with the inactive integrase virus carrying LV-TK or LV+TK. GFP virus with wild-type integrase and inactive integrase was used as a negative control since the GFP vector does not contain the sequence encoding Cas12a enzyme. Glyceraldehyde-3-phosphate dehydrogenase (GAPDH): housekeeping gene.

### EXAMPLE 1: Set-up of the experimental model

Given the prominent role of the *TP53* gene in cancer progression and drug resistance, the present inventors selected this experimental model to assess the efficacy and specificity of the CRISPR system of the invention. As previously described, the CRISPR system of the invention, through viral transduction, allows a gene encoding a cell death-promoting protein (for example, thymidine kinase, TK) to be inserted into a target cell, for example a tumour cell. Experiments carried out by the present inventors used tumour-specific *TP53* gene mutations as the genomic target of the CRISPR system according to the invention, demonstrating that the action of this system induces cell death only in tumour cells, without causing any damage to normal cells. Table 1 lists tumours associated with *TP53* gene mutations.

**Table 1. Percentage of TP53 mutations in different types of tumours.**

| **Other haematological tumours** | **% patients with TP53 mutation** | **Solid tumours** | **% patients with TP53 mutation** | **Solid tumours** | **% patients with TP53 mutation** |
|---|---|---|---|---|---|
| Non-Hodgkin's lymphoma | 66-11% | Squamous cell lung | 81.0% | Prostate | 17.5% |
| Myelodysplastic syndromes | 20-5% | Lung adenocarcinoma | 46.0% | Liver | 31.9% |
| Myeloproliferative neoplasms | 15% | Ovary | 47.8% | Brain | 27.0% |
| Multiple myeloma | 10% | Colon-rectum | 43.2% | Bladder | 26.4% |
| Acute myeloid leukemia | 5% | Esophagus | 43.1% | Breast | 25.0% |
| Acute lymphoblastic leukemia | 3% | Head and neck | 40.6% | Uterus | 20.5% |
| | | Larynx | 40.4% | Soft tissue | 19.1% |
| | | Skin | 35.0% | Bone tissue | 14.6% |
| | | Pancreas | 32.6% | Endocrine glands | 14.6% |
| | | Stomach | 32.0% | Cervix | 5.8% |

For experimental studies, a haematological tumour was selected, more specifically B-cell chronic lymphocytic leukemia. The CRISPR system according to the invention was first tested *in vitro* by using HEK293 and MCF7 cells engineered with the *TP53* gene target mutation (c.548C>G; p.Ser183*), and B-CLL cells carrying *TP53* gene mutations isolated from a patient.

In order to increase the specificity of the CRISPR system of the invention for B-CLL target cells, a pseudotyped HIV-derived lentiviral vector was designed and generated, which had lost its ability to integrate into the cell genome and was specifically targeted to B cells by using measles virus envelope glycoproteins and an anti-CD20/CD5 antibody (Funke S., et al, "Targeted cell entry of lentiviral vectors, (2008) Mol Ther. 16(8):1427-36). The various steps of the experimental approach followed by the present inventors are as described below.

### MATERIALS AND METHODS

### EXAMPLE 2: Isolation of peripheral blood mononuclear cells (PBMCs) and cell culture conditions

In order to prepare chronic lymphocytic leukemia lymphocytes, peripheral blood mononuclear cells (PBMCs) were isolated from leukemia patient blood samples. Blood samples were diluted with an equal volume of phosphate saline solution (pH 7.5) (PBS). The diluted blood was then carefully layered over a volume of human Pancoll (PAN Biotech) equal to the original blood sample volume. The sample was centrifuged at 350xg for 30 minutes at room temperature to obtain the formation of the PBMC layer. After centrifugation, the PBMC layer was transferred to 15 ml tubes with PBS. PBMCs were again centrifuged at 350xg for 20 minutes at room temperature. Subsequently, PBMCs were resuspended in complete growth medium.

HEK293 (ATCC CRL 3216), MCF7 (ATCC # HTB-22) and primary human fibroblast cells were maintained in Dulbecco's Modified Eagle's Medium (DMEM) (Invitrogen) with 10% heat-inactivated Fetal Bovine Serum (FBS) (Invitrogen) and 2mM L-glutamine (Invitrogen). Primary human fibroblasts were isolated from skin biopsies from voluntary donors and cultured with DMEM, 10% FBS, 2 mM L-glutamine, 50 units/ml penicillin (Invitrogen) and 50 g/ml streptomycin (Invitrogen). PBMCs were grown in Roswell Park Memorial Institute (RPMI) medium, 10% FBS and 4mM L-glutamine.

### EXAMPLE 3: Lentiviral construct, gRNA design and cloning

The lentivirus (LV) was purchased from Addgene and subsequently modified to insert the desired sequences essential for the correct functioning of the CRISPR system according to the invention. The first and second gRNA sequences were designed and selected by using the platform available on the Massachusetts Institute of Technology's (MIT) website (http://crispr.mit.edu). The nucleotide sequences encoding the first and second gRNAs consist of the sequence SEQ ID NO.2 (scaffold sequence: nucleotides (nt) 1-20; guide sequence: nt 21-43) and SEQ ID NO.3 (scaffold sequence: nt 1-20; guide sequence: nt 21-43), respectively. The nucleotide sequence of the second guide RNA, i.e. the gRNA whose target sequence is only present on the viral vector, was selected among sequences that are able to hybridize exclusively in the vicinity of the EGFP-HSV-TK sequence and not to other regions of the vector. The following sequences were inserted in the viral vector: the sequences encoding the first gRNA (SEQ ID NO.2) and the second gRNA (SEQ ID NO.3), the EGFP-HSV-TK cassette (SEQ ID NO.4), the sequence encoding Cas12a endonuclease (SEQ ID NO.5), the first target nucleotide sequence (SEQ ID NO.6) and the second target nucleotide sequence (SEQ ID NO.7). The following regulatory elements were also inserted: the U6 promoter sequence (SEQ ID NO.8), upstream of the sequences encoding the first and second gRNAs, as well as two PAM sequences, the first (5'-GATA-3') downstream of the first target sequence, and the second (5'-TATC-3') downstream of the second target sequence. The insertion of the aforementioned sequences was carried out by following a standard cloning protocol, typically comprising the following steps: cutting the DNA sequence with specific restriction enzymes, ligating the desired sequence, checking for the correct ligation, transforming in competent bacterial cells and extracting the plasmid DNA. EGFP-HSV-TK cassette was synthesized throughout and inserted in the lentiviral vector by using the KpnI restriction site. The nucleotide sequences of the gRNAs were initially synthesized as single-stranded DNA fragments and then phosphorylated and paired for subsequent insertion on the sides of the EGFP-HSV-TK cassette by using EcoRI (first gRNA) and BamHI (second gRNA) enzyme restriction sites. To confirm the correctness of the cloning, the DNA of the viral expression vector thus obtained was fully sequenced (sequence designated as SEQ ID NO.1).

### EXAMPLE 4: Recombinant viral vector DNA transformation, selection and isolation

The DNA of the recombinant viral vector was transformed in *E. coli* DH5α competent cells (Invitrogen) following the supplier's instructions and isolated by using the "EndoFree Plasmid" kit (Qiagen), following the vendor's standard instructions. Transformed cells were selected with ampicillin antibiotic. EGFP lentiviral vector (Addgene, no.21316) was used as a positive control for transduction.

### EXAMPLE 5: HEK293 and MCF-7 engineered with the TP53 gene mutation

Engineered HEK293 and MCF7 cell lines were obtained by randomly inserting a cassette including the specific TP53 gene mutation (c.548C>G; p.Ser183*) in their genome. The cassette with the point mutation (C>G) and the mCherry-bsr sequence was cloned into a plasmid as previously reported (Severi F. and Conticello SG. Flow-cytometric visualization of C > U mRNA editing reveals the dynamics of the process in live cells. RNA Biol. 2015; 12:389-397). The vector was transfected with lipofectamine 2000 (Invitrogen) into HEK293 and MCF7 cell lines by following the protocol illustrated in Example 3 section. Transfection efficiency was tested by mCherry expression.

### EXAMPLE 6: Viral transfection and transduction

1.2×10⁵ HEK293 and MCF7 cells, both also in the engineered form, were used for viral transfection and transduction experiments. The cells were cultured alone or co-cultured with primary fibroblasts and distributed in a 24-well plate. 24 hours later, the cells were Lipofectamine 2000-transfected (Invitrogen) with the lentiviral expression vector (2 µg total) or the EGFP lentiviral vector as a control by following the manufacturer's instructions. The cells were then analysed by flow cytometry and MTT cell proliferation assay 48 hours and 6 days post-transfection, respectively. In order to generate the HIV retroviral vector, approximately 5×10⁵ HEK293 cells were co-transfected by Lipofectamine 2000 with 200 ng of vesicular stomatitis virus G protein expression plasmid (VSV-G) and 1 µg of MLV GVPol expression plasmid. The supernatant containing the virus was collected, centrifuged and filtered 48 hours after transfection. A total of 5 x 10⁵ cells and 200 µl of virus was used for transduction in the presence of 5 µg/ml polybrene (Sigma).

### EXAMPLE 7: Off-target analysis

The present inventors carried out specific investigations in order to demonstrate lower exposure of the cells to Cas12a enzyme effects in the CRISPR system of the invention. A first investigation analysed the persistence of the lentiviral genome in HEK293T cells infected with the inactive integrase compared to cells infected with a wild-type integrase system. By using the GFP protein as the reporter of the system, this experiment demonstrated the almost complete clearance of the viral genome from the cell culture within one week after infection compared to 100% of GFP-expressing cells after 18 days of infection using the wild-type integrase (Figure 4). Cas12a expression mediated by the CRISPR system according to the invention was also compared to that mediated by an equivalent plasmid in which the target sequences leading to excision of the cassette had been removed, in the presence of mutated or wild-type integrase. The comparison shows a significant decrease in Cas12a expression in the CRISPR system object of the invention compared to the different control conditions (Figure 5).

### EXAMPLE 8: Flow cytofluorimetry analysis (FACS) and cell selection to verify Cas editing and detect clones derived from a single modified cell

The flow cytofluorimeter (BD) acquired 50,000 events/well. The data was analysed by FlowJo v7.5 cytofluorimetry software. For the analysis, HEK293 cells genetically modified with the CRISPR system of the invention were selected based on size and morphology; a second selection was then performed in order to detect GFP-positive cells in which the expression of the GFP protein is indicative of the correct integration of the HSV-TK sequence in the cell genome.

### EXAMPLE 9: In vitro cell analysis

The cells were distributed in triplicate in 48-well plates at a density of 0.8 × 10⁵ cells/well and transfected with and without HSV-TK lentivirus according to the invention. After 6 days, Ganciclovir (GCV), a thymidine kinase enzyme substrate, was administered to the cells at a concentration of 12µM and left in contact with the cells for 4 hours. The HSV-TK gene product phosphorylates GCV into GCV triphosphate, which is incorporated into the DNA during cell replication, thereby inhibiting DNA synthesis and causing cell death. Cells were subsequently analysed with 3-[4,5-Dimethylthiazol-2-yl]-2,5-diphenyltetrazolium bromide (MTT) to assess the percentage of cell viability after Ganciclovir addition and the mortality caused by administration of this compound. The MTT assay estimates the metabolic activity of the cells since the MTT assay is based on the conversion of the water soluble yellow MTT solution into insoluble purple crystals by living cells, which indicates mitochondrial activity.

### RESULTS

### EXAMPLE 10: Transfection of HEK293 and MCF7 cells.

HEK293 and MCF7 cell lines, specially engineered with the *TP53* gene mutations, were co-cultured with human fibroblasts to establish a heterogeneous cell population. HEK293 and MCF7 cells were transfected using lipofectamine with the lentiviral vector according to the invention carrying sequences encoding the first and second gRNAs, and the Cas12a and TK enzymes, respectively. In order to verify the successful transfection, the TK gene sequence was linked, through a 2A peptide sequence, to a marker, in particular to the sequence expressing the enhanced green fluorescent protein (EGFP). Accordingly, GFP-positive cells are also TK-positive. The lentiviral vector devoid of the EGFP-TK cassette and the lentiviral vector constitutively encoding the EGFP-TK sequence were used as controls in the transfection experiments.

The results of the *in vitro* experiments were analysed by FACS 3 days after transfection. Cells were recovered by FACS in order to select positively transfected cells based on the EGFP expression, proportional to the amount of cells in which the genome editing mediated by the CRISPR-Cas system of the invention was successful. The FACS analysis demonstrated high efficiency of the CRISPR-Cas system of the invention since a high percentage of cells positive for the marker signal was found.

In order to verify the correct integration of the TK gene coding sequence in the target genomic locus, the DNA extracted from the FACS-recovered cells was amplified by using a set of primers specific for the sequence integrated into the genomic locus. An amplicon of the expected 822-bp length was obtained only in amplification reactions performed on DNA extracted from engineered cell lines transfected with the lentiviral vector according to the invention, thus indicating the successful integration of the TK gene coding sequence into the genomic site carrying the specific target mutation.

Subsequently, using the engineered HEK293 and MCF7 cells, the wild-type HEK293 and MCF7 cells transfected as described above, experiments were carried out in order to verify the functionality of the TK-encoding gene upon integration into the target genomic site. Briefly, six days after transfection, EGFP-positive cells were selected by FACS. The recovered cells were incubated in the presence and the absence of Ganciclovir at a concentration of 0.1 mg/ml. From a subsequent FACS analysis carried out after 3 days of incubation, a cell death value of 70% was reported in the mutated HEK293 sample transfected with the CRISPR system of the invention, and an 80% reduction was detected in the mutated and wild-type HEK293 samples transfected with the lentiviral vector constitutively encoding the EGFP-TK sequence (Figure 2). Similar results were obtained with the MTT viability assay. In fact, the MTT colorimetric analysis revealed that a significant percentage of cells modified by the CRISPR system of the invention, compared to the control, died following treatment with ganciclovir, thus indicating that the TK-encoding sequence was correctly integrated into the target genomic locus and that the expressed enzyme is able to convert ganciclovir into a toxic compound harmful to cells.

### EXAMPLE 11: Culture and transduction of TP53-gene mutated and non-mutated leukemia lymphocytes

After the efficiency of the CRISPR system of the invention was confirmed in the analysed cell lines, the present inventors tested said system in primary cell lines. Lymphocytes isolated from blood samples from B-CLL patients, including both the leukemia lymphocytes carrying the specific *TP53* gene mutation, which represents the genomic target sequence of the first gRNA encoded by the lentiviral vector of the invention, and non-*TP53* mutated leukemia lymphocytes, were used as target cells. Since B lymphocytes are known from the literature to be difficult to transfect and transduce, electroporation was chosen as the method of transducing the lentiviral vector according to the invention into *TP53*-gene mutated and non-mutated leukemia B cells. Successful integration of the EGFP-TK cassette into the mutated genomic site exclusively in the lymphocyte population carrying the *TP53*-gene mutation was demonstrated by FACS analysis and DNA amplification, according to the methods illustrated in Example 8.

### EXAMPLE 12: Viral production and transduction

After the efficiency of the CRISPR system of the invention was demonstrated in *in vitro* cell lines, the present inventors generated viral particles capable of infecting target cells. Lentiviral particles (Addgene) were produced by following the standard viral production protocol indicated by the manufacturer. For this purpose, HEK293 cell line cells were transfected with a plasmid that enables viral protein packaging, a plasmid that drives the production of viral envelope proteins, and the lentiviral expression vector of the invention, together with a fourth plasmid encoding a modified Cas9 enzyme (dCas9) essential to avoid self-degradation of the lentiviral vector itself. In fact, in the absence of dCas9, the lentiviral vector, once inside the cell, expresses the guide RNAs and the Cas12a enzyme which, by being complexed into a macromolecular system, are able to excise the EGFP-TK cassette from the vector, resulting in the opening and degradation of the latter. On the other hand, in the presence of dCas9, which has lost its catalytic activity, this enzyme, guided by the gRNA molecules, binds to the target sequences on the vector preventing Cas12a from recognizing them. This endonuclease cannot therefore cut the double strand at target sites.

Alternatively, in order to avoid self-degradation of the lentiviral vector, a modified endonuclease (dCas12a) can be used. This strategy comprises transfecting a dCas12a-encoding plasmid into HEK293 cells, together with the plasmid that enables the packaging of viral proteins and a plasmid that drives the production of viral envelope proteins, both essential for the production of the virus. The role of the modified endonuclease is to bind to the guide RNA molecules, sequestering them, so that the operative Cas12a cannot target the target sites due to the low amount of free gRNAs available.

The present inventors employed the viral particles generated as described above to infect normal and *TP53*-gene mutated HEK293 and MCF7 cell lines. The presence of the correct integration of the EGFP-TK cassette in the target genomic site was verified by subjecting cell samples to FACS analysis 2 days after infection. The green fluorescence signals were localized exclusively in the target cells of the CRISPR system, i.e. in the mutated cells. These results were confirmed by amplifying genomic DNA extracted from the cells and detecting the presence of the expected 822-bp band only in virus-infected cells carrying the cassette with the mutation.

## Claims

1. A non-naturally occurring or engineered "Clustered Regularly Interspersed Short Palindromic Repeat (CRISPR)-CRISPR associated (Cas) "system (CRISPR system) targeting one genomic target sequence in a target cell, the system comprising a viral expression vector which comprises:
1) a nucleotide sequence encoding an endonuclease enzyme capable of generating cohesive ends, said nucleotide sequence being operably linked to one or more regulatory sequences located on the vector;
2) a nucleotide sequence encoding a protein capable of promoting cell death, said nucleotide sequence not being operably linked to any regulatory sequence located on the vector;
3) a first target nucleotide sequence and a second target nucleotide sequence located, respectively, downstream of the 3' end and upstream of the 5' end of the nucleotide sequence encoding the protein capable of promoting cell death, both the first target nucleotide sequence and the second target nucleotide sequence being located downstream of the 3' end of a Protospacer Adjacent Motif (PAM) nucleotide sequence;
4) a nucleotide sequence encoding a first guide RNA (first gRNA) operably linked to one or more regulatory sequences located on the vector, wherein said first gRNA comprises a first scaffold nucleotide sequence capable of binding the endonuclease enzyme and a first guide nucleotide sequence capable of hybridizing to the first target nucleotide sequence; and
5) a nucleotide sequence encoding a second guide RNA (second gRNA) operably linked to one or more regulatory sequences located on the viral expression vector, wherein said second gRNA comprises a second scaffold nucleotide sequence capable of binding the endonuclease enzyme and a second guide nucleotide sequence capable of hybridizing to the second target nucleotide sequence, wherein the first target nucleotide sequence corresponds to the genomic target sequence in the target cell targeted by the CRISPR system;
wherein the first target nucleotide sequence and the second target nucleotide sequence are different, and wherein the first target nucleotide sequence and the second target nucleotide sequence are selected so that the protruding single-stranded portions of the respective cohesive ends generated upon endonuclease enzyme cleavage are complementary sequences; and
wherein the genomic target sequence in the target cell targeted by the CRISPR system comprises at least one nonsense gene mutation.

2. The CRISPR system according to claim 1, wherein the endonuclease enzyme is selected from the group consisting of Cas12a and mutants thereof which are capable of binding to different PAM sequences.

3. The CRISPR system according to claim 1 or 2, wherein the protein capable of promoting cell death is the thymidine kinase enzyme.

4. The CRISPR system according to any of claims 1 to 3, wherein the gene mutation is selected from the group consisting of *TP53* gene mutations, *KRAS*/*BRAF* gene mutations, *NRAS* gene mutations, *EGFR* gene mutations in the region encoding the extracellular domain of the receptor, and *MET, PK3CA* and *ERB2* gene mutations, and any combination thereof.

5. The CRISPR system according to any of claims 1 to 4, wherein the target cell is a eukaryotic cell, preferably a human cell.

6. A viral particle comprising the CRISPR system according to any of claims 1 to 5.

7. The viral particle according to claim 6, which is selected from the group consisting of lentivirus, retrovirus, adenovirus and adeno-associated virus.

8. The viral particle according to claim 6 or 7, which comprises a chimeric capsid protein, said chimeric capsid protein comprising a domain capable of binding to at least one marker expressed on the surface of the target cell targeted by the CRISPR system,
wherein said domain is an antibody, a growth factor or a ligand, and/or
wherein said marker is selected from the group consisting of CD5, CD19, CD20, CD23, CD46, BCR protein, and any combination thereof.

9. An *in vitro* method for promoting apoptosis in a target cell, said method comprising the steps of:
- transducing the target cell with a CRISPR system according to any of claims 1 to 5 and/or with at least one viral particle according to any of claims 6 to 8, and
- culturing the transduced cell under suitable conditions for inducing the expression of the endonuclease enzyme and the first and second guide RNA (gRNA) and for obtaining the formation of a first macromolecular complex comprising the endonuclease enzyme associated with said first gRNA and a second macromolecular complex comprising the endonuclease enzyme associated with said second gRNA.

10. The method according to claim 9, wherein the protein capable of promoting cell death is the thymidine kinase enzyme.

11. The method according to claim 10, further comprising the step of contacting the target cell with a substrate of the thymidine kinase, preferably ganciclovir.

12. The method according to any of claims 9 to 11, wherein the target cell is a eukaryotic cell, preferably a human tumour cell.

13. An isolated target cell which comprises a CRISPR system according to any of claims 1 to 5 and/or at least one viral particle according to any of claims 6 to 8.

14. The CRISPR system according to any of claims 1 to 5 or the viral particle according to any of claims 6 to 8, for use as a medicament.

15. The CRISPR system or the viral particle according to claim 14, for use in the therapeutic treatment of a disease selected from the group consisting of B-cell Chronic Lymphocytic Leukemia, non-Hodgkin lymphoma, myelodysplastic syndromes, myeloproliferative neoplasms, multiple myeloma, acute myeloid leukemia, acute lymphoblastic leukemia, lung squamous cell carcinoma, lung adenocarcinoma, ovarian cancer, colorectal cancer, esophageal cancer, head and neck cancer, laryngeal cancer, skin cancer, pancreatic cancer, stomach cancer, prostate cancer, liver cancer, brain tumour, bladder cancer, breast cancer, uterine cancer, soft tissue sarcoma, bone cancer, endocrine tumours and cervical cancer.

## Patentansprüche

1. Nicht natürlich vorkommendes oder künstlich entwickeltes "gruppierte kurze palindromische Wiederholungen mit regelmäßigen Abständen und CRISPR associated (Cas)" System (CRISPR-System), das auf eine genomische Zielsequenz in einer Zielzelle zielt, wobei das System einen viralen Expressionsvektor umfasst, der enthält:
1) eine Nukleotidsequenz, die ein Endonuklease-Enzym codiert, das in der Lage ist, kohäsive Enden zu erzeugen, wobei diese Nukleotidsequenz funktionell mit einer oder mehreren regulatorischen Sequenzen, die sich auf dem Vektor befinden, verknüpft ist;
2) eine Nukleotidsequenz, die ein Protein codiert, das in der Lage ist, den Zelltod zu fördern, wobei diese Nukleotidsequenz nicht funktionell mit einer regulatorischen Sequenz, die sich auf dem Vektor befindet, verknüpft ist;
3) eine erste Ziel-Nukleotidsequenz und eine zweite Ziel-Nukleotidsequenz, die sich jeweils stromabwärts des 3'-Endes und stromaufwärts des 5'-Endes der Nukleotidsequenz befinden, die das Protein codiert, das in der Lage ist, den Zelltod zu fördern, wobei sowohl die erste Ziel-Nukleotidsequenz als auch die zweite Ziel-Nukleotidsequenz sich stromabwärts des 3'-Endes einer Protospacer Nachbarmotiv (PAM)-Nukleotidsequenz befinden;
4) eine Nukleotidsequenz, die eine erste Guide-RNA (erste gRNA) codiert, die funktionell mit einer oder mehreren regulatorischen Sequenzen, die sich auf dem Vektor befinden, verknüpft ist, wobei diese erste gRNA eine erste Gerüststruktur-Nukleotidsequenz umfasst, die in der Lage ist, an das Endonuklease-Enzym zu binden, sowie eine erste Guide-Nukleotidsequenz, die in der Lage ist, mit der ersten Ziel-Nukleotidsequenz zu hybridisieren; und
5) eine Nukleotidsequenz, die eine zweite Guide-RNA (zweite gRNA) codiert, die funktionell mit einer oder mehreren regulatorischen Sequenzen, die sich auf dem viralen Expressionsvektor befinden, verknüpft ist, wobei diese zweite gRNA eine zweite Gerüststruktur-Nukleotidsequenz umfasst, die in der Lage ist, an das Endonuklease-Enzym zu binden, sowie eine zweite Guide-Nukleotidsequenz, die in der Lage ist, mit der zweiten Ziel-Nukleotidsequenz zu hybridisieren, wobei die erste Ziel-Nukleotidsequenz der genomischen Zielsequenz in der Zielzelle entspricht, auf die das CRISPR-System zielt;
wobei die erste Ziel-Nukleotidsequenz und die zweite Ziel-Nukleotidsequenz verschieden sind, und wobei die erste Ziel-Nukleotidsequenz und die zweite Ziel-Nukleotidsequenz ausgewählt sind, sodass die hervorstehenden einzelsträngigen Abschnitte der jeweiligen kohäsiven Enden, die durch die Spaltung des Endonuklease-Enzyms erzeugt wurden, komplementäre Sequenzen sind; und
wobei die genomische Zielsequenz in der Zielzelle, auf die das CRISPR-System zielt, mindestens eine Nonsense-Genmutation umfasst.

2. CRISPR-System nach Anspruch 1, wobei das Endonuklease-Enzym aus der Gruppe bestehend aus Cas12a und dessen Mutanten, die in der Lage sind, an verschiedene PAM-Sequenzen zu binden, ausgewählt wird.

3. CRISPR-System nach Anspruch 1 oder 2, wobei das Protein, das in der Lage ist, den Zelltod zu fördern, das Enzym Thymidinkinase ist.

4. CRISPR-System nach einem der Ansprüche 1 bis 3, wobei die Genmutation aus der Gruppe bestehend aus Mutationen des TP53-Gens, Mutationen der KRAS/BRAF-Gens, Mutationen des NRAS-Gens, Mutationen des EGFR-Gens in der Region, die die extrazelluläre Domäne des Rezeptors codiert, sowie Mutationen der Gene MET, PK3CA und ERB2, und jede Kombination davon, ausgewählt wird.

5. CRISPR-System nach einem der Ansprüche 1 bis 4, wobei die Zielzelle eine eukaryotische Zelle ist, vorzugsweise eine menschliche Zelle.

6. Virales Partikel, das das CRISPR-System nach einem der Ansprüche 1 bis 5 umfasst.

7. Virales Partikel nach Anspruch 6, das aus der Gruppe bestehend aus Lentiviren, Retroviren, Adenoviren und Adeno-assoziierten Viren ausgewählt wird.

8. Virales Partikel nach Anspruch 6 oder 7, das ein chimäres Kapsidprotein umfasst, wobei dieses chimäre Kapsidprotein eine Domäne enthält, die in der Lage ist, an mindestens einen Marker zu binden, der auf der Oberfläche der Zielzelle exprimiert wird, auf die das CRISPR-System zielt, wobei diese Domäne ein Antikörper, ein Wachstumsfaktor oder ein Ligand ist, und/oder
wobei dieser Marker aus der Gruppe bestehend aus CD5, CD19, CD20, CD23, CD46, dem BCR-Protein und jeder Kombination davon, ausgewählt wird.

9. In vitro-Verfahren zum Fördern der Apoptose in einer Zielzelle, wobei dieses Verfahren die folgenden Schritte umfasst:
- Transduzieren der Zielzelle mit einem CRISPR-System nach einem der Ansprüche 1 bis 5 und/oder mit mindestens einem viralen Partikel nach einem der Ansprüche 6 bis 8, und
- Kultivieren der transduzierten Zelle unter geeigneten Bedingungen zur Induktion der Expression des Endonuklease-Enzyms sowie der ersten und zweiten Guide-RNA (gRNA) und zum Erhalten der Bildung eines ersten makromolekularen Komplexes umfassend das Endonuklease-Enzym, das der ersten gRNA zugeordnet ist, sowie eines zweiten makromolekularen Komplexes umfassend das Endonuklease-Enzym, das der zweiten gRNA zugeordnet ist.

10. Verfahren nach Anspruch 9, wobei das Protein, das in der Lage ist, den Zelltod zu fördern, das Enzym Thymidinkinase ist.

11. Verfahren nach Anspruch 10, ferner umfassend den Schritt, die Zielzelle mit einem Substrat der Thymidinkinase, vorzugsweise Ganciclovir, in Kontakt zu bringen.

12. Verfahren nach einem der Ansprüche 9 bis 11, wobei die Zielzelle eine eukaryotische Zelle ist, vorzugsweise eine menschliche Tumorzelle.

13. Isolierte Zielzelle, die ein CRISPR-System nach einem der Ansprüche 1 bis 5 und/oder mindestens ein virales Partikel nach einem der Ansprüche 6 bis 8 umfasst.

14. CRISPR-System nach einem der Ansprüche 1 bis 5 oder das virale Partikel nach einem der Ansprüche 6 bis 8 zur Verwendung als Arzneimittel.

15. CRISPR-System oder das virale Partikel nach Anspruch 14 zur Verwendung in der therapeutischen Behandlung einer Krankheit, die aus der Gruppe bestehend aus B-Zell-Chronischer Lymphatischer Leukämie, Non-Hodgkin-Lymphom, myelodysplastischen Syndromen, myeloproliferativen Neoplasien, Multiplem Myelom, Akuter Myeloischer Leukämie, Akuter Lymphoblastischer Leukämie, Plattenepithelkarzinom der Lunge, Adenokarzinom der Lunge, Eierstockkrebs, Darmkrebs, Speiseröhrenkrebs, Kopf-Hals-Krebs, Kehlkopfkrebs, Hautkrebs, Bauchspeicheldrüsenkrebs, Magenkrebs, Prostatakrebs, Leberkrebs, Hirntumor, Blasenkrebs, Brustkrebs, Gebärmutterkrebs, Weichteilsarkom, Knochenkrebs, endokrinen Tumoren und Gebärmutterhalskrebs ausgewählt wird.

## Revendications

1. Un système « Clustered Regularly Interspersed Short Palindromic Repeat (CRISPR)-CRISPR associated (Cas) » (système CRISPR) non naturel ou conçu artificiellement ciblant une séquence cible génomique dans une cellule cible, ledit système comprenant un vecteur d'expression viral qui comprend:
1) une séquence nucléotidique codant une enzyme endonucléase capable de générer des extrémités cohésives, ladite séquence nucléotidique étant fonctionnellement liée à une ou plusieurs séquences régulatrices situées sur le vecteur;
2) une séquence nucléotidique codant une protéine capable de favoriser la mort cellulaire, ladite séquence nucléotidique n'étant pas fonctionnellement liée à une séquence régulatrice située sur le vecteur;
3) une première séquence nucléotidique cible et une seconde séquence nucléotidique cible situées, respectivement, en aval de l'extrémité 3' et en amont de l'extrémité 5' de la séquence nucléotidique codant la protéine capable de favoriser la mort cellulaire, la première séquence nucléotidique cible et la seconde séquence nucléotidique cible étant toutes deux situées en aval de l'extrémité 3' d'une séquence nucléotidique Protospacer Adjacent Motif (PAM);
4) une séquence nucléotidique codant un premier ARN guide (premier gRNA) fonctionnellement liée à une ou plusieurs séquences régulatrices situées sur le vecteur, ledit premier gRNA comprenant une première séquence nucléotidique d'échafaudage capable de se lier à l'enzyme endonucléase et une première séquence nucléotidique guide capable de s'hybrider à la première séquence nucléotidique cible; et
5) une séquence nucléotidique codant un second ARN guide (second gRNA) fonctionnellement liée à une ou plusieurs séquences régulatrices situées sur le vecteur d'expression viral, ledit second gRNA comprenant une seconde séquence nucléotidique d'échafaudage capable de se lier à l'enzyme endonucléase et une seconde séquence nucléotidique guide capable de s'hybrider à la seconde séquence nucléotidique cible, où la première séquence nucléotidique cible correspond à la séquence cible génomique dans la cellule cible ciblée par le système CRISPR;
où la première séquence nucléotidique cible et la seconde séquence nucléotidique cible sont différentes, et où la première séquence nucléotidique cible et la seconde séquence nucléotidique cible sont sélectionnées de manière que les portions monocaténaires saillantes des extrémités cohésives respectives générées lors du clivage par l'enzyme endonucléase soient des séquences complémentaires; et
où la séquence cible génomique dans la cellule cible ciblée par le système CRISPR comprend au moins une mutation génétique non-sens.

2. Le système CRISPR selon la revendication 1, où l'enzyme endonucléase est sélectionnée parmi le groupe constitué par Cas12a et ses mutants capables de se lier à différentes séquences PAM.

3. Le système CRISPR selon la revendication 1 ou 2, où la protéine capable de favoriser la mort cellulaire est l'enzyme thymidine kinase.

4. Le système CRISPR selon l'une quelconque des revendications 1 à 3, où la mutation génétique est sélectionnée parmi le groupe constitué par les mutations du gène TP53, les mutations des gènes KRAS/BRAF, les mutations du gène NRAS, les mutations du gène EGFR dans la région codant le domaine extracellulaire du récepteur, ainsi que les mutations des gènes MET, PK3CA et ERB2, et toute combinaison de celles-ci.

5. Le système CRISPR selon l'une quelconque des revendications 1 à 4, où la cellule cible est une cellule eucaryote, de préférence une cellule humaine.

6. Une particule virale comprenant le système CRISPR selon l'une quelconque des revendications 1 à 5.

7. La particule virale selon la revendication 6, qui est sélectionnée parmi le groupe constitué par le lentivirus, le rétrovirus, l'adénovirus et le virus adéno-associé.

8. La particule virale selon la revendication 6 ou 7, qui comprend une protéine de capside chimérique, ladite protéine de capside chimérique comprenant un domaine capable de se lier à au moins un marqueur exprimé à la surface de la cellule cible ciblée par le système CRISPR, ledit domaine étant un anticorps, un facteur de croissance ou un ligand, et/ou
où ledit marqueur est sélectionné parmi le groupe constitué par CD5, CD19, CD20, CD23, CD46, la protéine BCR, et toute combinaison de ceux-ci.

9. Un procédé in vitro pour favoriser l'apoptose dans une cellule cible, ledit procédé comprenant les étapes de:
- transduire la cellule cible avec un système CRISPR selon l'une quelconque des revendications 1 à 5 et/ou avec au moins une particule virale selon l'une quelconque des revendications 6 à 8, et
- cultiver la cellule transduite dans des conditions appropriées pour induire l'expression de l'enzyme endonucléase et du premier et du second ARN guide (gRNA) et pour obtenir la formation d'un premier complexe macromoléculaire comprenant l'enzyme endonucléase associée audit premier gRNA et d'un second complexe macromoléculaire comprenant l'enzyme endonucléase associée audit second gRNA.

10. Le procédé selon la revendication 9, où la protéine capable de favoriser la mort cellulaire est l'enzyme thymidine kinase.

11. Le procédé selon la revendication 10, comprenant en outre l'étape de mise en contact de la cellule cible avec un substrat de la thymidine kinase, de préférence le ganciclovir.

12. Le procédé selon l'une quelconque des revendications 9 à 11, où la cellule cible est une cellule eucaryote, de préférence une cellule tumorale humaine.

13. Une cellule cible isolée qui comprend un système CRISPR selon l'une quelconque des revendications 1 à 5 et/ou au moins une particule virale selon l'une quelconque des revendications 6 à 8.

14. Le système CRISPR selon l'une quelconque des revendications 1 à 5 ou la particule virale selon l'une quelconque des revendications 6 à 8, pour une utilisation en tant que médicament.

15. Le système CRISPR ou la particule virale selon la revendication 14, pour une utilisation dans le traitement thérapeutique d'une maladie sélectionnée parmi le groupe constitué par la leucémie lymphoïde chronique des cellules B, le lymphome non hodgkinien, les syndromes myélodysplasiques, les néoplasmes myéloprolifératifs, le myélome multiple, la leucémie myéloïde aiguë, la leucémie lymphoblastique aiguë, le carcinome épidermoïde pulmonaire, l'adénocarcinome pulmonaire, le cancer de l'ovaire, le cancer colorectal, le cancer de l'œsophage, le cancer de la tête et du cou, le cancer du larynx, le cancer de la peau, le cancer du pancréas, le cancer de l'estomac, le cancer de la prostate, le cancer du foie, la tumeur cérébrale, le cancer de la vessie, le cancer du sein, le cancer de l'utérus, le sarcome des tissus mous, le cancer des os, les tumeurs endocrines et le cancer du col de l'utérus.
